# EUROPÄISCHE PATENTSCHRIFT

(11) **EP 1 034 775 B1**
(45) Veröffentlichungstag und Bekanntmachung des Hinweises auf die Patenterteilung: **14.11.2001**
(21) Anmeldenummer: 00102980.0
(22) Anmeldetag: 14.02.2000
(51) Int. Cl.: A61K 7/06, A61K 7/50

(54) **Haarwaschmittel**
Hair-wash
Agent de lavage pour cheveux

(30) Priorität: 04.03.1999 DE 19909552
(43) Veröffentlichungstag der Anmeldung: 13.09.2000
(73) Patentinhaber: GOLDWELL GmbH, 64297 Darmstadt (DE)
(72) Erfinder: Heinz, Dieter, 65462 Gustavsburg (DE); Förster, Sabine, 64319 Pfungstadt (DE); Hoffmann, Martin, 64673 Zwingenberg (DE); Schup, Bettina, 64219 Pfungstadt (DE)

(56) Entgegenhaltungen:
- EP-A- 0 875 236
- EP-A- 0 998 906
- DE-A- 19 613 567
- DE-U- 29 605 428
- US-A- 4 676 978
- US-A- 5 573 756
- US-A- 5 993 792

## Beschreibung

Die Erfindung betrifft ein hautmildes, gut schäumendes Haarwaschmittel mit guten konditionierenden Eigenschaften.

Haarwaschmittel, vorwiegend solche auf Basis anionischer Tenside, werden seit Menschengedenken benutzt.
Seit etwa zwanzig Jahren enthalten solche Haarwaschmittel auch Wirkstoffe, die das Haar konditionieren, d.h., die Naß- und Trockenkämmbarkeit, das Volumen, die Fülle, den Griff und den Glanz des gewaschenen Haares verbessern sollen.
Bevorzugt eingesetzte konditionierende Wirkstoffe sind dabei natürliche oder synthetische Polymere sowie quaternäre langkettige Ammoniumverbindungen, wobei bei letzteren allerdings Kompatibilitätsprobleme mit anionischen Tensiden auftreten können.

Diese Haarwaschmittel auf Basis anionischer Tenside können, je nach Charakter und Menge des anionischen Tensids, bei empfindlichen Benutzern in seltenen Fällen Haut- und Schleimhautreizungen hervorrufen, weshalb in manchen Fällen auf spezielle Tenside ausgewichen wird, was das Shampoo jedoch erheblich verteuert.

Die vorliegende Erfindung geht von der Aufgabenstellung aus, ein Haarwaschmittel auf wäßriger Basis, enthaltend anionische und nichtionische Tenside, zu schaffen, das eine sehr gute Hautverträglichkeit und ausgezeichnete konditionierende Eigenschaften besitzt, ohne irgendwelche Stabilitätsprobleme aufzuweisen.

Diese Aufgabe wird erfindungsgemäß durch ein flüssiges Haarwaschmittel gelöst, das 5 bis 50 Gew.-%, berechnet auf seine Gesamtzusammensetzung, eines Tensidgemisches, umfassend eine Kombination aus
a) mindestens einem anionischen Tensid, vorzugsweise in einer Menge von 2,5 bis 25Gew.-% der Gesamtzusammensetzung, und
b) mindestens einem nichtionischen Tensid, vorzugsweise in einer Menge von 2,5 bis 25 Gew.-% der Gesamtzusammensetzung, sowie
c) 0,05 bis 5 Gew.-% mindestens eines kationischen Polymeren, und
   Aus der US-A-4676978 ist ein haarkonditionierendes Shampoo bekannt, das anionische waschaktive Substanzen, ein polykationisches Guar-Derivat, ein wasserlösliches härtbares Polykondensationsprodukt aus einem Polyamin und einem Polyalkylenglykol mit terminalen Halohydrin- oder Hydroxylgruppen und anschließender Reaktion mit Epichlorhydrin, einen Polyethylenglykolglycerinfettsäureester und Polyvinylpyrrolidon enthält.
   Die US-A-5573756 beschreibt konditionierende Shampoos auf Basis anionaktiver Tenside, quaternären Ammoniumsalzen und einem Polyethylenglykolträger, die in Form von Kapseln vorliegen.
d) 0,1 bis 10 Gew.-% mindestens eines Polyoxyethylenpropandiolmono- und/oder -di-C₁₀-C₂₄-fettsäureesters enthält.

Diese Zusammensetzungen sind stabil, sehr gut haut- und schleimhautverträglich, schäumen gut und verleihen dem Haar eine ausgezeichnete Naß- und Trockenkämmbarkeit, Glanz, Fülle, Volumen und einen vollen, weichen Griff.

Geeignete anionaktive Tenside im Rahmen der Erfindung sind in einer Menge von vorzugsweise mindestens 5 bis 25 Gew.-% der Zusammensetzung enthalten.

Dabei handelt es sich um solche vom Sulfat-, Sulfonat-, Carboxylat- und Alkylphosphat-Typ, vor allem natürlich diejenigen, die in Shampoos üblicherweise zum Einsatz gelangen, beispielsweise die bekannten C₁₀-C₁₈-Alkylsulfate und insbesondere die entsprechenden Ethersulfate, beispielsweise C₁₂-C₁₄-Alkylethersulfat, Laurylethersulfat, insbesondere mit 1 bis 4 Ethylenoxidgruppen im Molekül, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettige Mono- und Dialkylphosphate, die milde, hautverträgliche Detergentien darstellen.

Im Rahmen der Erfindung weiterhin geeignete anionische Tenside sind α-Olefinsulfonate bzw. deren Salze und insbesondere Alkalisalze von Sulfobernsteinsäurehalbestern, beispielsweise das Dinatriumsalz des Monooctylsulfosuccinats und Alkalisalze langkettiger Monoalkylethoxysulfosuccinate.

Geeignete Tenside vom Carboxylat-Typ sind Alkylpolyethercarbonsäuren und deren Salze der Formel

R-(C₂H₄O)ₙ-O-CH₂COOX,

worin R eine C₈-C₂₀-Alkylgruppe, vorzugsweise C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel Gegenstand der Erfindung ist weiterhin die Verwendung von Polyoxyethylenpropandiolmono- und/oder -di-C₁₀-C₂₄-fettsäureestern zur Verbesserung der Hautverträglichkeit von wäßrigen Haarwaschmittein, die 5 bis 50 Gew.-% eines Tensidgemisches aus mindestens einem anionischen und mindestens einem nichtionischen Tensid sowie mindestens 0,05 bis 5 Gew.-% mindestens eines kationischen Polymeren, jeweils berechnet auf die Gesamtzusammensetzung des Mittels, enthalten. 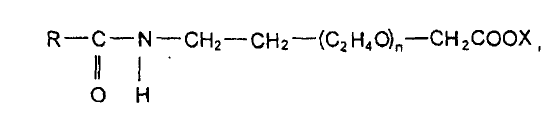 worin R und X die vorstehend angegebene Bedeutung haben, und n insbesondere für eine Zahl von 1 bis 10, vorzugsweise 2,5 bis 5, steht.

Derartige Produkte sind seit längerem bekannt und im Handel, beispielsweise unter den Handelnamen "AKYPO® " und AKYPO-SOFT® ".

Auch C₈-C₂₀-Acylisethionate können, allein oder im Gemisch mit anderen anionaktiven Tensiden, eingesetzt werden, ebenso Sulfofettsäuren und deren Ester.
Es können auch Mischungen aus mehreren anionischen Tensiden verwendet werden, beispielsweise ein Gemisch aus einen α-Olefinsulfonat und einem Sulfosuccinat, vorzugsweise im Verhältnis 1:3 bis 3:1, oder einem Ethersulfat und einer Polyethercarbonsäure oder Alkylamidoethercarbonsäure.
Eine Übersicht über die in flüssigen Körperreinigungsmitteln zum Einsatz gelangenden anionaktiven Tenside findet sich im übrigen in der Monographie von K. Schrader, "Grundlagen und Rezepturen der Kosmetika", 2. Aufl.(1989, Hüthig Buchverlag), S. 595-600 und S. 683 bis 691.

Weitere geeignete anionische Tenside sind auch C₈-C₂₂-Acylaminocarbonsäuren bzw. deren wasserlöslichen Salze, vorzugsweise in einer Menge von 0,5 bis 10, insbesondere 1 bis 7,5 Gew.-%, berechnet auf die Gesamtzusammensetzung. Besonders bevorzugt sind N-Lauroylglutamat, insbesondere als Natriumsalz, sowie beispielsweise N-Lauroylsarcosinat, N-C₁₂-C₁₈-Acylasparaginsäure, N-Myristoylsarcosinat, N-Oleoylsarcosinat, N-Lauroylmethylalanin, N-Lauroyllysin und N-Lauroylaminopropylglycin, vorzugsweise in Form ihrer wasserlöslichen Alkali- oder Ammonium-, insbesondere Natriumsalze, vorzugsweise im Gemisch mit den obengenannten anionaktiven Tensiden.

Weitere essentielle Tenside in den erfindungsgemäßen Haarwaschmitteln sind nichtionische Tenside im Gemisch mit anionaktiven und gegebenenfalls zwitterionischen bzw. amphoteren Tensiden.

Diese sind bei Schrader, l.c., auf den Seiten 600-601 und S. 694-695 beschrieben. Geeignet sind insbesondere Alkylpolyglucoside mit der allgemeinen Formel

R-O-(R¹O)ₙ-Zₓ,

worin R eine Alkylgruppe mit 8 bis 18 Kohlenstoffatomen, R¹ eine Ethylen- oder Propylengruppe, Z einen Saccaridrest mit 5 bis 6 Kohlenstoffatomen, n eine Zahl von 0 bis 10 und x eine Zahl zwischen 1 und 5 bedeuten.
Diese Alkylpolyglucoside sind in letzter Zeit insbesondere als ausgezeichnete hautverträgliche schaumverbessernde Mittel in flüssigen Wasch- und Körperreinigungsmitteln bekannt geworden und sind in einer Menge von etwa 2,5 bis 25, insbesondere 5 bis 10 Gew.-%, der Gesamtzusammensetzung enthalten.

Gemische aus anionaktiven Tensiden und Alkylpolyglucosiden sowie deren Verwendung in flüssigen Körperreinigungsmitteln sind an sich bereits bekannt, beispielsweise aus der EP-A 70 074. Die dort beschriebenen Alkylpolyglucoside sind prinzipiell auch im Rahmen der vorliegenden Erfindung geeignet; ebenso die aus der EP-A 358 216 bekannten Gemische aus Sulfosuccinaten und Alkylpolyglucosiden.

Weitere nichtionische Tensidbestandteile sind beispielsweise langkettige Fettsäuremono- und dialkanolamide, wie Cocosfettsäuremonoethanolamid und Myristinfettsäuremonoethanolamid, die auch als Schaumverstärker, vorzugsweise in einer Menge von 1 bis 5 Gew.-% eingesetzt werden können.

Andere zusätzlich mitverwendbare nichtionische Tenside sind z.B. die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind, sowie Fettalkoholethoxylate. Weitere zusätzlich einsetzbare Tenside sind Aminoxide in einer Menge von 0,25 bis 5, vorzugsweise 0,5 bis 3,5 Gew.-%, berechnet auf die Gesamtzusammensetzung des Mittels.

Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder -ethylaminoxide, C₁₂-C₁₈-Alkyldi(hydroxyethyl)- oder -(hydroxypropyl)aminoxide, oder auch Aminoxide mit Ethylenoxid- und/oder Propylenoxidgruppen in der Alkylkette. Solche Aminoxide sind beispielsweise unter den Bezeichnungen "Ammonyx® ", "Aromox® " oder "Genaminox® " im Handel.

Die erfindungsgemäßen Zusammensetzungen können als weiteren Tensid-Bestandteil amphotere bzw. zwitterionische Tenside, beispielsweise in einer Menge von 0,5 bis 10, vorzugsweise von 1 bis 7,5 Gew.-%, bezogen auf die Gesamtzusammensetzung, enthalten.

Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoacetat, Cocoaminopropionat und Natriumcocoamphopropionat und -acetat haben sich als geeignet erwiesen. Im einzelnen können Betaine der Struktur 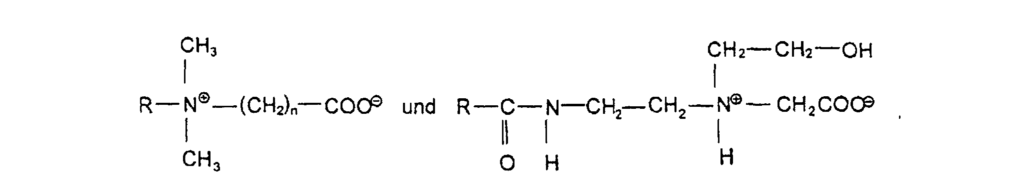
wobei R eine C₈-C₁₈-Alkylgruppe und n 1 bis 3 bedeuten,
   Sulfobetaine der Struktur 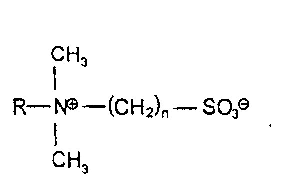
wobei R eine C₈-C₁₈-Alkylgruppe und n 1 bis 3 bedeuten,
   und Amidoalkylbetaine der Struktur 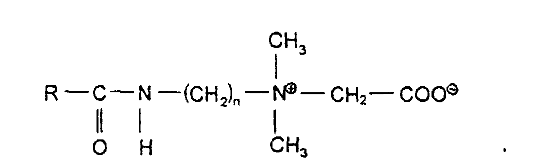
wobei R eine C₈-C₁₈-Alkylgruppe und n 1 bis 3 bedeuten, verwendet werden.

Bevorzugte haarkonditionierende kationische Polymere sind die altbekannten quaternären Cellulosederivate des Typs "Polymer JR" sowie quaternisierte Homo- und Copolymere des Dimethyldiallylammoniumchlorids, wie sie unter dem Handelsnamen "Merquat® " im Handel sind, quaternäre Vinylpyrrolidon-Copolymere, insbesondere mit Dialkylaminoalkyl(meth)-acrylaten, wie sie unter dem Namen "Gafquat® " bekannt sind, Copolymerisate aus Vinylpyrrolidon und Vinylimidazoliniummethochlorid, die unter dem Handelsnamen "Luviquat® " angeboten werden, Polyamino-Polyamid-Derivate, beispielsweise Copolymere von Adipinsäure-Dimethylaminohydroxypropyldiethylentriamin, wie sie unter dem Namen "Cartaretine® F" vertrieben werden, sowie auch bisquaternäre langkettige Ammoniumverbindungen der in der US-PS 4 157 388 beschriebenen Harnstoff-Struktur, die unter dem Handelsnamen "Mirapol® A 15" im Handel sind.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es könne auch Mischungen verschiedener kationischer Polymerer eingesetzt werden.

Zu den kationischen Polymeren zählen auch die in der EP-A 524 612 und der EP-A 640 643 beschriebenen Quaternisierungsprodukte aus Pfropfpolymerisaten von Organopolysiloxanen und Polyethyloxazolinen.

Der bevorzugte Anteil an kationischen Polymeren liegt bei 0,1 bis 3, insbesondere 0,25 bis 2,5 Gew.-%, berechnet auf die Gesamtzusammensetzung des Shampoos.

Der Polyoxyethylenpropandiolmono- und/oder difettsäureester ist vorzugsweise in einer Menge von 0,25 bis 5, insbesondere 0,5 bis 2,5 Gew.-%, berechnet auf die Gesamtzusammensetzung des Shampoos, enthalten.

Bevorzugt ist ein Polyoxyethylen-(30-60)-1,2-propandiolester mit einer C₁₂-C₁₈-Fettsäure, beispielsweise PEG-55-propylenglykolmono- und-dioleat; jedoch sind auch andere Ester wie beispielsweise PEG-8-propylenglykolcocoat, PEG-25-propylenglykolstearat und PEG-75-propylenglykolstearat geeignet.
Diese Verbindungen sind an sich bekannt und im Handel erhältlich.

Gemäß einer bevorzugten Ausführungsform der Erfindung enthalten die Zusammensetzungen noch etwa 0,25 bis 10 Gew.-%, vorzugsweise etwa 0,5 bis 5 Gew.-% eines Polyoxyethylenglycerylmono, -di- und -trifettsäureesters, vorzugsweise einer C₁₀-C₂₂-, insbesondere C₁₂-C₁₈-Fettsäure.

Besonders geeignet sind PEG-(15-30)-glycerylfettsäureester, z.B. PEG-15-glyceryloleat, PEG-18-glyceryloleat, PEG-18-glycerylcocoat, PEG-30-glycerylcocoat, PEG-25-glycerylcocoat, PEG-20-glyceryllaurat, PEG-30-glycerlylisostearat, PEG-30-glyceryllaurat, PEG-25-glyceryloleat, PEG-20-glycerylindioleat, PEG-10-glycerylstearat, PEG-25-glycerylstearat, PEG-28-glyceryltallowat, PEG-25-glyceryldi bzw. -trioleat, etc., jedoch sind auch entsprechende Glycerylfettsäureester mit weniger als 15 bzw. mehr als 30 Ethylenoxidgruppen geeignet, z.B. PEG-7-glycerylcocoat, PEG-40-glycerylcocoat, PEG-12-glyceryldioleat und PEG-5-glycerylstearat.

Die erfindungsgemäßen Shampoos können auch weitere Wirkstoffe wie Eiweißhydrolysate und Polypeptide, z.B. Keratinhydrolysate, Kollagenhydrolysate vom Typ "Nutrilan® " oder Elastinhydrolysate sowie insbesondere auch pflanzliche, gegebenenfalls kationisierte Eiweißhydrolysate, z.B. "Gluadin® ", enthalten.

Auch der Zusatz weiterer konditionierender Polymerer ist möglich.
Diese können nichtionische Polymere, vorzugsweise alkohol- und/oder wasserlösliche Vinylpyrrolidon-Polymere wie ein Vinylpyrrolidon-Homopolymerisat oder -Copolymerisat, insbesondere mit Vinylacetat, sein.
Geeignete Vinylpyrrolidon-Polymere sind z.B. die unter dem Handelsnamen "Luviskol® " bekannten Produkte, beispielsweise die Homopolymerisate "Luviskol® K 30, K 60 und K 90" sowie die wasser- bzw. alkohollöslichen Copolymerisate aus Vinylpyrrolidon und Vinylacetat, die unter dem Handelsnamen "Luviskol® VA 55 bzw. VA 64" von der BASF AG vertrieben werden.

Weitere geeignete nichtionische Polymere sind Vinylpyrrolidon/Vinylacetat/Vinylpropionat-Copolymere wie "Luviskol® VAP 343", Vinylpyrrolidon/(Meth)Acrylsäureester-Copolymere sowie Chitosan-Derivate. Ihr Anteil in den erfindungsgemäßen Haarwaschmitteln liegt, wenn vorhanden, zwischen 0,05 und 5, vorzugsweise 0,1 und 2,5, insbesondere 0,15 bis 1,5 Gew.-%, bezogen auf die Gesamtzusammensetzung des Mittels.

Amphotere Polymere, die im Gemisch mit mindestens einem kationischen Polymeren zum Einsatz gelangen, sind insbesondere Copolymerisate aus N-Octylacrylamid, (Meth)-Acrylsäure und tert.-Butylaminoethylmethacrylat vom Typ "Amphomer® "; Copolymerisate aus Methacryloylethylbetain und Alkylmethacrylaten vom Typ "Yukaformer® ", z.B. das Butylmethacrylat-Copolymere "Yukaformer® Am 75"; Copolymerisate aus Carboxylgruppen und Sulfongruppen enthaltenden Monomeren, z.B. (Meth)Acrylsäure und Itaconsäure, mit basischen Gruppen, insbesondere Aminogruppen, enthaltenden Monomeren wie Mono- bzw. Dialkylaminoalkyl(meth)acrylaten bzw. Mono- bzw. Dialkylarninoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der US-A 3,927,199 bekannten Copolymeren.

Falls erwünscht, können zusätzlich zu den obligatorischen kationischen Polymeren auch anionische Polymere mitverwendet werden.

Geeignete anionische Polymere sind Vinylalkylether-, insbesondere Methylvinylether/ Maleinsäure-Copolymere, die durch Hydrolyse von Vinylether/Maleinsäureanhydrid-Copolymeren entstehen und unter der Handelbezeichnung "Gantrez® AN oder ES" vertrieben werden. Diese Polymere können auch teilverestert sein, beispielsweise "Gantrez® ES 255", der Ethylester eines Ethylvinylethers/Maleinsäure-Copolymers, oder der Butyl- oder Isobutylester desselben.

Weitere geeignete anionische Polymere sind insbesondere Vinylacetat/Crotonsäure- oder Vinylacetat/Vinylneodecanoat/Crotonsäure-Copolymere des Typs "Resyn® ", Natriumacrylat/Vinylalkohol-Copolymere des Typs "Hydagen® F", Natriumpolystyrolsulfonat, z.B."Flexan® 130", Ethylacrylat/Acrylsäure/N-tert.-Butylacrylamid-Copolymere des Typs"Ultrahold® ", Vinylpyrrolidon/Vinylacetat/Itaconsäure-Copolymere, Acrylsäure/ Acrylamid-Copolymere bzw. Natriumsalze derselben vom Typ "Reten® ".

Die erfindungsgemäßen Haarwaschmittel können selbstverständlich alle üblichen, in solchen Mitteln zum Einsatz gelangenden Stoffe enthalten.

Als solche seien beispielhaft Komplexbildner, Farbstoffe, Konservierungsmittel, pH-Regler, Viskositätsregler wie anorganische Salze, soweit sie nicht ohnehin in den Tensid-Ausgangsmischungen enthalten sind, Duftstoffe, Perlglanzmittel, Verdickungsmittel, Feuchthaltemittel, pflanzliche und tierische Öle, etc. genannt.

Geeignete pflanzliche und tierische Öle sowie synthetische Fettsäureester, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweie Silikonöle, Polyethylenglykole, etc. Weitere geeignete hydrophobe Komponenten sind insbesondere Fettalkohole, vorzugsweise soclhe mit 8 bis 22 Kohlenstoffatomen im Molekül wie Myristyl-, Cetyl-, Stearylalkohol,Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat und Cetylpalmitat.

Diese hydrophoben Komponenten sind in den erfindungsgemäßen Zusammensetzungen vorzugsweise in einer Gesamtmenge von 0,5 bis 10, insbesondere 1 bis 7,5, vor allem 1,5 bis 5 Gew.-%, berechnet auf die Gesamtzusammensetzung, enthalten.

Eine Auflistung solcher Zusatzstoffe findet sich ebenfalls bei Schrader, l.c., auf S. 695 bis 722.

Weitere bevorzugte Bestandteile sind Benzylalkohol, Benzyloxyethanol, Phenoxyethanol, Phenethylalkohol, Zimtalkohol, 1-Methoxypropanol(-2), 1-Ethoxypropanol(-2), Diethylenglykolmonomethyl- oder -ethylether, Dipropylenglykolmonomethyl- oder -ethylether und/ oder Harnstoff, vorzugsweise in einer Menge von 0,1 bis 10 Gew.-%, insbesondere 0,5 bis 7,5 Gew.-% des erfindungsgemäßen Körperreinigungsmittels.

Schließlich können auch noch bekannte Polysiloxane als konditionierende Mittel in den erfindungsgemäßen flüssigen Haarwaschmitteln mitverwendet werden. Deren bevorzugter Anteil liegt dabei zwischen 0,5 und 5, insbesondere 1 bis 3 Gew.-% der Gesamtzusammensetzung. Geeignet sind sowohl leichtflüchtige als auch schwerflüchtige cyclische oder lineare Polysiloxane, beispielsweise die unter dem Trivialnamen "Dimethicone" bzw. "Phenyldimethicone" sowie "Cyclomethicone" bekannten Silikonöle.

Geeignet sind beispielsweise auch die in der EP-A 398 177 beschriebenen Silikonderivate, die dort in Kombination mit Alkylpolyglucosiden in flüssigen Detergens-Zusammensetzungen eingesetzt werden.

Die erfindungsgemäßen Shampoos können auch Farbstoffe zur direkten oder oxidativen Färbung von Haaren enthalten, also sogenannte Tönungs- oder Färbeshampoos sein. Besonders geeignet sind direktziehende Farbstoffe enthaltende Tönungsshampoos.

Die Viskosität liegt im üblichen Bereich zwischen 1000 und 10 000 mPa.s bei 20°C, gemessen nach Brookfield oder Höppler bei einer Scherspannung von 10 sec⁻¹.
Sie können, abgefüllt mit entsprechenden Treibmitteln, auch als Aerosolshampoos konfektioniert werden.
Der pH-Wert der erfindungsgemäßen Shampoos liegt im üblichen Bereich zwischen 5 und 8,5, für Spezialprodukte kann er auch unterhalb 5 eingestellt werden.

Die folgenden Beispiele dienen der Illustration der Erfindung.

### Beispiel 1

| **Shampoo für normales Haar** | |
|---|---|
| Natriumlaurylethersulfat (∼2,5EO) | 8,5 (Gew.-%) |
| C₁₂-C₁₄-Alkylpolyglucosid (P.D.:∼1,5) | 3,0 |
| Natriumlaurylethersulfosuccinat | 1,0 |
| Polyquaternium-10 | 0,2 |
| PEG-55-1,2-propylenglykololeat | 1,0 |
| PEG-18-glyceryloleat/cocoat | 1,0 |
| PEG-160-sorbitantriisostearat | 0,3 |
| Parfum | 0,5 |
| Pflanzenextrakte | 0,6 |
| Panthenol | 0,3 |
| Natriumchlorid | 1,8 |
| Milchsäure | 0,1 |
| Citronensäure | 0,2 |
| 2-Pyrrolidon-5-carbonsäure | 0,1 |
| Benzylalkohol | 0,5 |
| 1,2-Propandiol | 0,5 |
| Glycerin | 0,5 |
| Konservierungsmittel | 0,3 |
| Wasser | ad 100,0 |

Dieses Shampoo wurde in "Occlusive Multiple Patch Test" mit einem identischen Shampoo verglichen, in dem die Bestandteile PEG-55-1,2-propylenglykololeat/PEG-18-glycerylcocoat/-oleat durch die gleiche Menge Wasser ersetzt waren.

Für das erfindungsgemäße Shampoo wurde ein Irritation Score-Wert von 0,48 ermittelt, während das Vergleichsshampoo einen solchen von 0,84 aufwies.
Das bedeutet, daß durch den erfindungsgemäßen Zusatz des Polyoxyethylenpropandiolfettsäureesters das Reizpotential des Shampoos in etwa halbiert wurde.

Das Shampoo nach Beispiel 1 entwickelte bei der Anwendung mit Wasser einen vollen, cremigen Schaum und verlieh dem Haar eine gute Naß- und Trockenkämmbarkeit, Volumen, vollen und weichen Griff sowie Glanz und Spannkraft.

### Beispiel 2

| **Shampoo für feines Haar** | |
|---|---|
| Natriumalkylethersulfat (∼2,5EO) | 6,5 (Gew.-%) |
| C₁₂-C₁₄-Alkylpolyglucosid (P.D.:∼1,5) | 2,9 |
| Cocoamidopropylbetain | 1,8 |
| Laurylhydroxysultain | 1,0 |
| Natriumlauroylglutamat | 1,1 |
| PEG-35-1,2-propylenglykolcocoat | 2,0 |
| PEG-3-distearat | 0,6 |
| Triglycerin | 0,2 |
| 1,2-Propandiol | 1,0 |
| Natriumchlorid | 1,2 |
| Kation. Weizenproteinhydrolysat | 0,3 |
| Polyquaternium-6 | 0,3 |
| Panthenol | 0,3 |
| Aloeextrakt | 0,1 |
| Natriumisostearoyllactylat | 0,1 |
| Parfum | 0,5 |
| Konservierungsmittel | 0,3 |
| Wasser | ad 100,0 |

Dieses Shampoo entsprach hinsichtlich Schaumvermögen und haarkonditionierenden Eigenschaften im wesentlichen demjenigen nach Beispiel 1.
Der "Irritation Score" lag bei 0,45; ein identisch zusammengesetztes Vergleichsprodukt, jedoch ohne PEG-35-1,2-propandiolcocoat, ergab einen deutlich schlechteren Wert von 0,73.

### Beispiel 3

| **Antifett-Shampoo** | |
|---|---|
| Natriumlaurylethersulfat (∼2 EO) | 10,5 (Gew.-%) |
| C₁₂-C₁₄-Alkylpolyglucosid | 2,5 |
| Polyoxyethylen-(55)-1,2-propandioldioleat | 2,0 |
| Polyquaternium-10 | 0,3 |
| Natriumchlorid | 2,2 |
| 1,2-Propandiol | 1,0 |
| Allantoin | 0,3 |
| Panthenol | 0,3 |
| Isopropylmyristat | 0,5 |
| PEG-3-distearat | 0,8 |
| PEG-20-glyceryltallowat | 0,5 |
| Thymianextrakt | 0,5 |
| Parfum | 0,5 |
| Citronensäure | 0,3 |
| Milchsäure | 0,1 |
| Glykolsäure | 0,1 |
| Äpfelsäure | 0,1 |
| Glycerin | 1,0 |
| Natriumisostearyllactylat | 0,3 |
| Konservierungsmittel | 0,3 |
| Wasser | ad 100,0 |

Die Schaumqualität und die haarkonditionierenden Eigenschaften dieses Shampoos entsprechen im wesentlichen denjenigen des Beispiels 1.
Der "Irritation Score" wurde mit 0,40 ermittelt; ein Shampoo ohne Polyoxyethylen-(55)-propylenglykoldioleat wies einen Wert von 0,72 auf.

### Beispiel 4

| **Antischuppen-Shampoo** | |
|---|---|
| Natriumlaurylethercarbonsäure (∼2,5EO) | 8,0 (Gew.-%) |
| Dinatriumlaurylethersulfosuccinat | 1,0 |
| C₁₂-C₁₄-Alkylpolyglucosid (P.D.:∼1,5) | 2,5 |
| Cocoamidopropylbetain | 2,0 |
| Polyoxyethylen-(50)-1,2-propandiolmono/distearat | 1,5 |
| PEG-28-glyceryltallowat | 0,5 |
| Polyquaternium-11 | 0,4 |
| 1,2-Propylenglykol | 0,6 |
| Natriumchlorid | 1,0 |
| Triglycerin | 0,5 |
| Weizenproteinhydrolysat | 0,5 |
| Parfum | 0,5 |
| Panthenol | 0,3 |
| Piroctone Olamine | 0,4 |
| Benzylalkohol | 0,5 |
| Pflanzenextrakte | 0,5 |
| Konservierungsmittel | 0,3. |
| Wasser | ad 100,0 |

Neben einer ausgezeichneten Antischuppenwirkung und exzellentem Schaumvermögen zeigte dieses Shampoo gute haarkonditionierende Eigenschaften.
Der "Irritation Score" für dieses Shampoo wurde mit 0,46 bestimmt, gegenüber 0,84 für ein identisches Produkt ohne Polyoxyethylenpropylenglykolfettsäureester.

### Beispiel 5

| **Shampoo für poröses und trockenes Haar** | |
|---|---|
| Natriumlaurylethersulfat (∼2,5 EO) | 8,0 (Gew.-%) |
| C₁₂-C₁₄-Alkylpolyglucosid (P.D.:∼1,5) | 2,5 |
| Dinatriumlaurylethersulfosuccinat | 2,0 |
| Laurylhydroxysultain | 1,5 |
| Polyquaternium-4 | 0,4 |
| Triglycerin | 0,5 |
| PEG-40 hydriertes Ricinusöl | 0,5 |
| PEG-3-distearat | 0,5 |
| Isostearoylactylat | 0,5 |
| Glycerin | 0,5 |
| 1,2-Propandiol | 0,8 |
| Polyoxyethylen-55-propylenglykolmono/dioleat | 1,0 |
| Polyoxyethylen-25-propylenglykolmonostearat | 0,8 |
| PEG-160-diisostearat | 1,0 |
| Weizenproteinhydrolysat | 0,7 |
| Weinsäure | 0,1 |
| Äpfelsäure | 0,1 |
| Milchsäure | 0,1 |
| Glykolsäure | 0,1 |
| Citronensäure | 0,2 |
| 2-Pyrrolidon-5-carbonsäure | 0,1 |
| Benzylalkohol | 1,0 |
| Panthenol | 0,3 |
| Deceth-8 | 0,5 |
| Parfum | 0,5 |
| Konservierungsmittel | 0,3 |
| Wasser | ad 100,0 |

Neben guten Schaumeigenschaften zeigte das Shampoo eine sehr gute konditionierende Wirkung auf geschädigtem Haar.
Der "Irritation Score" lag bei 0,36; Weglassen des Gemisches aus PEG-55-propylenglykolmono/dioleat und PEG-25-propylenglykolmonostearat führte zu einem Shampoo mit einem "Irritation Score" von 0. 8.

## Patentansprüche

1. Haarwaschmittel auf wäßriger Basis, enthaltend 5 bis 50 Gew.-%, berechnet auf die Gesamtzusammensetzung, eines Tensidgemisches, umfassend eine Kombination aus
a) mindestens einem anionischen Tensid,
b) mindestens einem nichtionischen Tensid;
c) 0,05 bis 5 Gew.-% mindestens eines kationischen Polymeren, und
d) 0,1 bis 10 Gew.-% mindestens eines Polyoxyethylenpropandiolmono- und/oder -di-C₁₀-C₂₄⁻Fettsäureesters.

2. Mittel nach Anspruch 1, enthaltend 2,5 bis 25 Gew.-%, berechnet auf die Gesamtzusammensetzung, des Bestandteils a).

3. Mittel nach Anspruch 1 oder 2, enthaltend 2,5 bis 25 Gew.-%, berechnet auf die Gesamtzusammensetzung, des Bestandteils b).

4. Mittel nach einem oder mehreren der Ansprüche 1 bis 3, enthaltend als Bestandteil b) ein C₈-C₁₈-Alkylpolyglucosid der allgemeinen Formel
R-O-(R¹O)ₙ-Zₓ,
worin R eine Alkylgruppe mit 8 bis 18 Kohlenstoffstomen, R¹ eine Ethylen- oder Propylengruppe, Z einen Saccharidrest mit 5 bis 6 Kohlenstoffatomen, n eine Zahl von 0 bis 10, und x eine Zahl zwischen 1 und 5 bedeuten.

5. Mittel nach einem oder mehreren der Ansprüche 1 bis 4, enthaltend zusätzlich ein zwitterionisches und/oder amphoteres Tensid.

6. Mittel nach Anspruch 5, enthaltend 0,5 bis 10 Gew.-% eines zwitterionischen und/oder amphoteren Tensids, berechnet auf die Gesamtzusammensetzung.

7. Mittel nach einem oder mehreren der Ansprüche 1 bis 6, enthaltend 0,25 bis 5 Gew.-% des Bestandteils d).

8. Mittel nach Anspruch 6, enthaltend als Bestandteil d) 0,5 bis 2,5 Gew.-% Polyoxyethylen-(30-60)-1,2-propandiolmono- und/oder -dioleat.

9. Haarwaschmittel nach einem oder mehreren der Ansprüche 1 bis 8, enthaltend zusätzlich etwa 0,25 bis etwa 10 Gew.-%, berechnet auf die Gesamtzusammensetzung, mindestens eines Polyoxyethylenglycerylfettsäureesters.

10. Haarwaschmittel nach Anspruch 9, enthaltend 0,5 bis 5 Gew.-%, berechnet auf die Gesamtzusammensetzung, Polyoxyethylen-(15-30)-glyceryloleat und/oder -cocoat.

11. Verwendung von Polyoxyethylenpropandiolmono- und/oder -di-C₁₀-C₂₄-fettsäureestern zur Verbesserung der Hautverträglichkeit von wäßrigen Haarwaschmitteln, die 5 bis 50 Gew.-% eines Tensidgemisches aus mindestens einem anionischen und mindestens einem nichtionischen Tensid sowie mindestens 0,05 bis 5 Gew.-% mindestens eines kationischen Polymeren, jeweils berechnet auf die Gesamtzusammensetzung des Mittels, enthalten.

## Claims

1. Shampoo composition on aqueous basis, containing 5 % to 50 % by weight, calculated to the total composition, of a surfactant mixture comprising a combination of
a) at least one anionic surfactant,
b) at least one nonionic surfactant;
c) 0.05 % to 5 % by weight of at least one cationic polymer, and
d) 0.1 % to 10 % by weight of at least one polyoxyethylene propanediol mono-and/or -di-C₁₀-C₂₄-fatty acid ester.

2. Composition according to claim 1, containing 2.5 % to 25 % by weight, calculated to the total composition, of component a).

3. Composition according to claim 1 or 2, containing 2.5 % to 25 % by weight, calculated to the total composition, of component b).

4. Composition according to one or more of claims 1 to 3, containing as component b) a C₈-C₁₈-alkyl polyglucoside of the general formula
R-O-(R¹O)ₙ-Zₓ,
wherein R is an alkyl group with 8 to 18 carbon atoms, R¹ is an ethylene or propylene group, Z is a saccharide group with 5 to 6 carbon atoms, n is a number from 0 to 10, and x is a number between 1 and 5.

5. Composition according to one or more of claims 1 to 4, containing in addition a zwitterionic and/or amphoteric surfactant.

6. Composition according to claim 5, containing 0.5 % to 10 % by weight of a zwitterionic and/or amphoteric surfactant, calculated to the total composition.

7. Composition according to one or more of claims 1 to 6, containing 0.25 % to 5 % by weight of component d).

8. Composition according to claim 6, containing as component d) 0.5 % to 2.5 % by weight of polyoxyethylene-(30-60)-1.2-propanediol mono- and/or dioleate.

9. Shampoo composition according to one or more of claims 1 to 8, containing in addition about 0.25 % to about 10 % by weight, calculated to the total composition, of at least one polyoxyethylene glyceryl fatty acid ester.

10. Shampoo composition according to claim 9, containing 0.5 % to 5 % by weight, calculated to the total composition, of polyoxyethylene-(15-30)-glyceryl oleate and/or cocoate.

11. Use of polyoxyethylene propanediol mono- and/or di-C₁₀-C₂₄-fatty acid esters to improve the skin compatibility of aqueous shampoo compositions, which contain 5 % to 50 % by weight of a surfactant mixture of at least one anionic and at least one nonionic surfactant, as well as at least 0.05 % to 5 % by weight of at least one cationic polymer, each calculated to the total composition.

## Revendications

1. Agent de lavage dés cheveux à base aqueuse, contenant de 5 à 50 % en poids, calculés par rapport à la composition totale, d'un mélange d'agents tensio-actifs comprenant une combinaison de:
a) au moins un agent tensio-actif anionique,
b) au moins un agent tensio-actif non ionique;
c) de 0,05 à 5 % en poids d'au moins un polymère cationique, et
d) de 0,1 à 10 % en poids d'au moins un ester de mono et/ou de diacide gras en C₁₀ à C₂₄ et de polyoxyéthylène propanediol.

2. Agent selon la revendication 1, contenant de 2,5 à 25 % en poids, calculés par rapport à la composition totale, du composant a).

3. Agent selon la revendication 1 ou 2, contenant de 2,5 à 25 % en poids, calculés par rapport à la composition totale, du composant b).

4. Agent selon l'une ou plusieurs des revendications 1 à 3, contenant comme composant b) un (alkyle C₈ à C₁₈) polyglucoside de formule générale:
R-O- (R¹O)ₐ-Zₓ
dans laquelle R représente un groupe alkyle comptant de 8 à 18 atomes de carbone, R¹ un groupe éthylène ou propylène, Z un radical saccharide contenant de 5 à 6 atome de carbone, n un nombre de 0 à 10 et x un nombre compris entre 1 et 5.

5. Agent selon une ou plusieurs des revendications 1 à 4, contenant en outre un agent tensio-actif zwitterionique ou amphotère.

6. Agent selon la revendication 5, contenant de 0,5 à 10 % en poids, calculés par rapport à la composition totale, de l'agent tensio-actif zwitterionique ou amphotère.

7. Agent selon l'une ou plusieurs des revendications 1 à 6, contenant de 0,25 à 5 % en poids du composant d).

8. Agent selon la revendication 6, contenant comme composant d) de 0,5 à 2,5 % en poids de mono ou de dioléate de polyoxyéthylène-(30-60)-1,2-propanediol.

9. Agent de lavage de cheveux selon l'une ou plusieurs des revendications 1 à 8, contenant en outre d'environ 0,25 à environ 10 % en poids, calculés par rapport à la composition totale, d'au moins un ester d'acide gras et de polyoxyéthylèneglycéryle.

10. Agent de lavage de cheveux selon la revendication 9, contenant de 0,5 à 5 % en poids, calculés par rapport à la composition totale, d'oléate et/ou de cocoate de polyoxyéthylène-(15-30)-glycéryle.

11. Utilisation d'esters de mono et/ou de diacide gras en C₁₀ à C₂₄ et de polyoxyéthylènepropanediol pour l'amélioration de la compatibilité avec la peau d'agents aqueux de lavage des cheveux qui contiennent de 5 à 50 % en poids d'un mélange d'agents tensio-actifs constitués d'au moins un agent tensio-actif anionique et d'au moins un agent tensio-actif non ionique, ainsi que d'au moins 0,05 à 5 % en poids d'au moins un polymère cationique, tous les pourcentages étant calculés par rapport à la composition totale de l'agent.
